# FASCICULE DE BREVET EUROPEEN

(11) **EP 2 852 372 B1**
(45) Date de publication et mention de la délivrance du brevet: **11.07.2018**
(21) Numéro de dépôt: 13730317.8
(22) Date de dépôt: 21.05.2013
(51) Int. Cl.: A61K 8/19, A61K 8/26, A61K 8/29, A61K 8/88, A61Q 19/08, A61K 8/02

(54) **COMPOSITION COSMETIQUE CONTENANT UNE DISPERSION DE PARTICULES A BASE DE POLYMERE ET DE CHARGES MINERALES**
KOSMETISCHE ZUSAMMENSETZUNG MIT EINER DISPERSION AUS POLYMERTEILCHEN UND MINERALISCHEN FÜLLSTOFFEN
COSMETIC COMPOSITION CONTAINING A DISPERSION OF POLYMER PARTICLES AND MINERAL FILLERS

(30) Priorité: 22.05.2012 FR 1254657
(43) Date de publication de la demande: 01.04.2015
(73) Titulaire: Rhodia Poliamida E Especialidades Ltda, CEP-05804-902 Sao Paulo (BR)
(72) Inventeur: CANOVA, Thomas, Gonzaga, Sao Paolo (BR); GORESCU, Gabriel, Sao Paulo (BR); CORDEIRO BASTOS, Tarcis, Sao Paulo (BR)
(74) Mandataire: Casalonga
(86) Numéro de dépôt international: PCT/IB2013/000989
(87) Numéro de publication internationale: WO 2013/175286

(56) Documents cités:
- EP-A1- 2 402 387
- US-A- 4 999 243
- US-A1- 2007 141 095
- US-B1- 6 316 102
- DATABASE WPI Week 200768 Thomson Scientific, London, GB; AN 2007-724646 XP002692151, & KR 2007 0006549 A (SAMSINSA CO LTD) 11 janvier 2007 (2007-01-11)
- DATABASE WPI Week 200712 Thomson Scientific, London, GB; AN 2007-119332 XP002692152, & KR 2006 0081174 A (AMOREPACIFIC CORP) 12 juillet 2006 (2006-07-12)

## Description

La présente invention a pour objet une composition cosmétique, qui comprend une dispersion de particules d'une composition polymérique dans laquelle sont dispersées plusieurs charges minérales lesquelles possèdent des propriétés d'absorption et/ou d'émission de radiations dans le domaine de l'infrarouge lointain.

La présente invention a également pour objet l'utilisation d'une telle composition pour prévenir ou diminuer les signes du vieillissement cutané, et en particulier pour lutter contre les rides.

On sait que l'apparence de la peau humaine évolue au cours du temps, en raison de phénomènes de vieillissements naturels, qui peuvent être accélérés par des facteurs extérieurs tels que la pollution, le mode de vie (par exemple l'alimentation, le stress, le tabagisme).

Ce vieillissement de la peau se traduit en particulier par l'apparition à sa surface de diverses marques telles que des rides plus ou moins profondes, des taches dites « taches de vieillesse ».

Ces marques de vieillissement cutané sont de plus en plus considérées comme inesthétiques, en particulier celles situées sur les parties apparentes du corps telles que le visage, le cou, les mains.

Ainsi, de nombreux produits cosmétiques ont été développés pour prévenir l'apparition des signes du vieillissement cutané et/ou diminuer ceux déjà installés. Ces produits se présentent en général sous la forme de compositions qui sont des fluides plus ou moins épais tels que des crèmes, des lotions, des sérums, et qui contiennent un ou plusieurs agents actifs antirides, dispersés ou dissous dans un fluide de base aqueux et/ou organique. Ces agents actifs sont des composés chimiques ou naturels destinés à lutter contre les rides et/ou les tâches. Ces produits cosmétiques doivent en général être appliqués sur les zones à traiter une à deux fois par jour, et présentent des degrés d'efficacité très variables.

La demande de brevet KR 2007 0006549 décrit une composition cosmétique destinée entre autres à réduire les rides de la peau, et qui contient des charges minérales émettrices de radiations infrarouge directement dispersées dans la composition.

Malgré les nombreux produits actuellement présents sur le marché, le consommateur est toujours à la recherche de solutions innovantes et efficaces, pouvant être utilisées à la place ou en complément des solutions déjà existantes. Il existe ainsi un besoin de proposer de nouvelles solutions, qui permettent de lutter de manière efficace contre le vieillissement de la peau.

Poursuivant ses recherches dans ce domaine, la Demanderesse a maintenant découvert une approche nouvelle et originale, qui permet de combattre efficacement les signes du vieillissement cutané, en particulier les rides et les tâches de vieillesse.

Cette approche repose sur l'utilisation d'une composition polymérique particulière, comprenant une matrice de polymère au sein de laquelle sont dispersées des charges minérales qui émettent et/ou absorbent des radiations infrarouges dans la plage de longueur d'onde située entre 2 µm et 20 µm.

Cette composition polymérique est dispersée sous forme de particules dans le fluide de base d'une composition cosmétique destinée à être appliquée sur la peau.

En effet la Demanderesse a découvert, de manière totalement inattendue, que l'application sur la peau d'une telle dispersion de particules formées de ladite composition polymérique avait pour effet de diminuer les signes de vieillissement déjà présents sur la peau, et de prévenir ou retarder l'apparition de nouveaux signes.

La présente invention a donc pour objet une composition cosmétique, comprenant des particules d'une composition polymérique laquelle contient une matrice polymérique et au moins deux charges minérales de types différents choisis parmi les oxydes, les sulfates, les carbonates, les phosphates et les silicates, uniformément dispersées dans la matrice polymérique, ayant des propriétés d'absorption et/ou d'émission dans la région d'infrarouge lointain allant de 2 µm à 20 µm, lesdites particules de composition polymérique étant dispersées au sein d'un fluide porteur comprenant de l'eau et/ou un ou plusieurs fluides organiques.

La présente invention a également pour objet une méthode de traitement cosmétique de la peau, consistant à mettre la peau au contact d'une composition cosmétique telle que décrite dans la présente demande.

La présente demande a enfin pour objet l'utilisation d'une telle composition cosmétique pour prévenir et/ou diminuer les signes du vieillissement de la peau.

De manière connue en soi, on désigne par « signes du vieillissement de la peau » les marques présentes sur la peau résultant des phénomènes de vieillissement, qui modifient son aspect visuel et sont généralement considérées comme inesthétiques, telles qu'en particulier les rides et les tâches de vieillesse.

La composition cosmétique selon l'invention présente une excellente efficacité pour lutter contre de tels signes du vieillissement cutané.

Elle présente en outre l'avantage d'être particulièrement douce lors de son application sur la peau, et non irritante pour celle-ci. En particulier, l'incorporation des charges minérales au sein d'une composition polymérique rend la composition plus douce, et moins abrasive pour la peau, que si ces mêmes charges étaient directement dispersées dans le fluide porteur.

Enfin, la dispersion des charges minérales dans le fluide porteur est plus homogène, et plus stable dans le temps, lorsque ces charges sont incorporées dans des particules de composition polymérique conformément à la présente invention.

L'invention met en oeuvre une composition polymérique comprenant une matrice polymérique.

La matrice polymérique peut être choisie en particulier dans le groupe comprenant : les polyesters, les polyoléfines, les polymères à base de cellulose-ester tels que l'acétate de cellulose, le propionate de cellulose, le rayon, la viscose et les polymères de la même famille, les polymères et copolymères acryliques, les polyamides tels que le polyhexaméthylène adipamide (PA66), le polycaproamide (PA6), les PA6.10, PA10.10 et PA12, les copolymères en toutes proportions de ces polymères, et les mélanges entre n'importe lesquels de ces polymères.

Selon une forme préférentielle de réalisation, la matrice polymérique est constituée de polyamide, de préférence choisi entre le polyamide 6, le polyamide 66 et les copolymères de polyamide 6/polyamide 66 en toutes proportions.

La composition polymérique selon l'invention comprend plusieurs charges minérales ayant des propriétés d'absorption et/ou d'émission dans la région d'infrarouge lointain allant de 2 à 20 µm. De préférence, la ou les charges minérales ont des propriétés d'absorption et/ou d'émission dans la région d'infrarouge lointain allant de 3 à 20 µm, et encore plus préférentiellement de 3 à 15 µm.

Selon l'invention, les charges minérales sont dispersées uniformément dans la matrice polymérique. Par « uniformément dispersées » on entend que les charges minérales sont incorporées de manière homogène au sein même du polymère. En particulier, les particules sont piégées dans la composition de polymère. Il ne s'agit donc pas de charges minérales déposées sur le polymère, par exemple sous la forme d'un revêtement à la surface du polymère (« coating »).

Une telle dispersion uniforme peut être obtenue en incorporant la ou les charges minérales dans le polymère lors de la synthèse de ce dernier. Un mode de réalisation consiste à réaliser une ou plusieurs suspension(s) de charges minérales, stabilisée(s) par des tensioactifs. La (les) suspension(s) est(sont) ensuite ajoutée(s) au cours de la synthèse du polymère.

L'on peut également incorporer lesdites charges par mélange de celles-ci au polymère fondu, soit directement, soit au moyen d'un concentré de particules sous forme de mélange maître (« masterbatch »), celui-ci pouvant être postérieurement dilué en concentrations prédéterminées dans la masse polymérique.

Grâce à de tels procédés l'on peut obtenir des compositions polymériques selon l'invention qui contiennent la ou les charges minérales de façon uniformément dispersée dans la matrice polymérique. Les charges minérales utilisables selon l'invention sont choisies parmi les oxydes, les sulfates, les carbonates, les phosphates et les silicates.

De préférence, le ou les oxyde(s) est (sont) choisi(s) parmi le dioxyde de titane, le dioxyde de silicium et l'oxyde de magnésium.

Le ou les sulfate(s) peuvent être avantageusement choisi(s) parmi les sulfates de métaux alcalins et de métaux alcalino-terreux, de préférence parmi le sulfate de baryum, le sulfate de calcium et le sulfate de strontium.

Le ou les carbonate(s) est (sont) choisi(s) avantageusement parmi le carbonate de calcium et le carbonate de sodium.

De préférence, le ou les silicate(s) est(sont) choisi(s) parmi l'actinolite, la tourmaline, la serpentine, le kaolinite, et le silicate de zirconium.

Le ou les phosphate(s) peuvent être choisi(s) parmi les phosphates de zirconium, le phosphate de cérium, l'apatite et leurs mélanges.

De manière préférée, la composition polymérique contient au moins trois charges minérales de types différents, choisis parmi les types énoncés ci-avant.

Selon un premier mode de réalisation préféré, la composition polymérique contient au moins deux charges minérales de types différents, choisis parmi les types suivants : les oxydes, les sulfates, et les silicates, et de préférence parmi le dioxyde de titane, un sulfate de métal alcalin ou alcalino-terreux et un silicate, et de manière encore plus préférée parmi le dioxyde de titane, le sulfate de baryum, et la tourmaline.

De manière plus préférée, la composition polymérique contient au moins trois charges minérales de types différents choisis parmi les types ci-avant. De manière particulièrement préférée, la composition polymérique contient trois charges minérales de types différents, qui sont un oxyde, un sulfate, et un silicate.

On préfère tout particulièrement l'association dioxyde de titane/sulfate de métal alcalino-terreux/silicate; et encore plus préférentiellement l'association dioxyde de titane/ sulfate de baryum/ tourmaline.

Dans ce cas, les proportions respectives en poids des trois charges minérales ci-avant sont de préférence comprises entre 80:10:10 et 10:30:60, et plus spécifiquement ces proportions respectives sont de 50:25:25.

Selon un second mode de réalisation, également avantageux, la composition polymérique contient au moins deux charges minérales de types différents, et de préférence au moins trois charges minérales de types différents choisies parmi les types suivants : les oxydes, les phosphates, et les silicates.

Dans ce mode de réalisation, on préfère en particulier les associations de trois charges minérales de types différents, à savoir un oxyde, un phosphate, et un silicate.

De préférence, la proportion en poids de charge(s) minérale(s) par rapport au poids total de la composition polymérique est supérieure ou égale à 1,0 %, de préférence supérieure ou égale à 1,5 % et plus préférentiellement encore supérieure ou égale à 2,5 %.

De préférence, la proportion en poids de charge(s) minérale(s) par rapport au poids total de la composition polymérique est inférieure ou égale à 50 %, de préférence inférieure ou égale à 40 %, et plus préférentiellement encore inférieure ou égale à 30 %. Les charges minérales selon l'invention se présentent avantageusement sous forme de particules, lesquelles présentent de préférence une taille moyenne en volume inférieure ou égale à 2 µm, mesurée selon la méthode d'analyse granulométrique par diffraction laser (en utilisant, par exemple, des granulomètres MALVERN ou CILAS).

Une manière avantageuse de procéder consiste à mettre les particules en suspension dans l'eau, et à déterminer leur granulométrie par diffraction laser en employant la méthode décrite dans la norme ISO 13320 :2009.

Il est préférable que les charges minérales utilisées dans la présente invention présentent une taille de particules qui ne soit :
- ni trop petite, pour prévenir tout risque que les particules ne puissent sortir de la matrice polymérique et s'introduire dans le corps humain au travers de la peau ou via les voies respiratoires, ou encore se disperser dans l'environnement ;
- ni trop grosse, ce qui rendrait plus difficile l'incorporation des particules dans la matrice polymérique et surtout pourrait rendre la composition cosmétique abrasive au contact de la peau, ce qui pourrait à terme avoir un effet irritant sur la peau, par exemple dans le cas de peaux particulièrement fines ou sensibles.

Ainsi, les charges minérales selon l'invention se présentent sous forme de particules qui présentent avantageusement une taille moyenne en volume, mesurée selon la méthode d'analyse granulométrique par diffraction laser, allant de 0,1 à 2 µm, plus préférentiellement de 0,2 à 1,5 µm et encore plus préférentiellement de 0,2 à 1 µm.

Les charges minérales présentent avantageusement une distribution granulométrique avec 99 % en volume des particules ayant une taille inférieure à 1,0 µm, de préférence 90 % en volume des particules ayant une taille inférieure à 0,5 µm. La distribution granulométrique est également mesurée par la méthode d'analyse granulométrique par diffraction laser précitée (en utilisant, par exemple, des granulomètres MALVERN ou CILAS).

La composition polymérique selon l'invention présente de préférence un nombre de pics d'absorption de radiations infrarouges supérieur à 10 dans les dix plages de fréquence suivantes : 3,00 +/-0,30µm, 6,20 +/- 0,50µm, 8,00 +/- 0,25µm, 8,50 +/- 0,25µm, 9,00 +/-0,25µm, 9,50 +/- 0,25µm, 10,00 +/- 0,25µm, 10,50 +/- 0,25µm, 11,00 +/- 0,25µm, 14,60 +/- 2,10µm, au moins 1 pic étant présent dans au moins 7 de ces dix plages de fréquence.

Le spectre d'absorption de radiations infrarouges peut être déterminé par toute méthode connue de l'homme du métier. Une méthode possible est l'utilisation d'un appareil Bruker Equinox 55, avec une résolution de 4 cm⁻¹. Dans ce cas le spectre obtenu est sous forme ATR (« Atenuated Total Reflectance »), en utilisant un cristal ZnSe.

Comme cela a été exposé ci-avant, la composition selon l'invention contient des particules d'une composition polymérique.

Ces particules de composition polymérique sont présentes en une teneur qui peut aller de 0,5 à 20% en poids, de préférence de 1 à 15% en poids, et plus préférentiellement de 2 à 10% en poids, par rapport au poids total de la composition cosmétique.

Ces particules de composition polymérique peuvent présenter toute forme et toute taille compatible avec une incorporation et une dispersion au sein d'un fluide porteur dans une composition cosmétique destinée à être appliquée sur la peau.

Selon un premier mode de mise en oeuvre préféré de l'invention, les particules de composition polymérique présentent une forme sensiblement sphérique, c'est-à-dire que les particules présentent une forme assimilable à celle d'une sphère, qui peut être plus ou moins régulière, par exemple ovalisée et/ou aplatie.

Dans ce mode de mise en oeuvre, les particules de composition polymérique présentent avantageusement une taille moyenne en volume inférieure ou égale à 250 µm, de préférence allant de 5 à 150 µm, et plus préférentiellement de 10 à 50 µm.

La taille moyenne en volume des particules de composition polymérique est mesurée selon la méthode d'analyse granulométrique par diffraction laser précitée (en utilisant, par exemple, des granulomètres MALVERN ou CILAS).

Dans ce mode de mise en oeuvre, le ratio entre la taille moyenne en volume des particules de composition polymérique et la taille moyenne en volume des charges minérales peut être également optimisé pour éviter tout risque que les particules, trop petites, ne puissent sortir de la matrice polymérique et s'introduire dans le corps humain ou se disperser dans l'environnement, ou au contraire ne soient trop grosses, avec le risque de rendre la composition abrasive au contact de la peau.

Ainsi, le ratio entre la taille moyenne en volume des particules de composition polymérique selon l'invention et la taille moyenne en volume des charges minérales, ces deux tailles étant mesurées selon la méthode d'analyse granulométrique par diffraction laser précitée, est avantageusement supérieur ou égal à 5. Ce ratio est de préférence inférieur ou égal à 250. Ce ratio va de préférence de 5 à 150, plus préférentiellement de 5 à 100.

Les particules de composition polymérique selon l'invention peuvent être préparées par les méthodes connues de l'homme du métier pour l'obtention de poudres ou fines particules de polymères, par exemple par broyage, cryo-broyage ou séchage par pulvérisation (« spray drying ») de la composition polymérique. L'on peut employer de manière alternative la méthode décrite dans la demande de brevet FR 2 899 591 au nom de la Demanderesse.

Selon un second mode de mise en oeuvre préféré de l'invention, les particules de composition polymérique présentent la forme de fibres, dont la longueur moyenne est de préférence inférieure ou égale à 3 mm, plus préférentiellement inférieure ou égale à 2 mm, encore plus préférentiellement inférieure ou égale à 1,5 mm.

Ces fibres présentent de préférence un diamètre moyen équivalent allant de 4 à 50 µm, de préférence de 6 à 30 µm et plus préférentiellement de 6 à 20 µm.

Ces deux paramètres (la longueur moyenne, et le diamètre moyen équivalent des fibres) sont avantageusement mesurés par microscopie optique.

Dans ce second mode de mise en oeuvre, le ratio entre la taille de la ou des charges minérales et le diamètre des fibres peut être également optimisé pour éviter tout risque que les particules, trop petites, ne puissent sortir de la matrice polymérique et s'introduire dans le corps humain ou se disperser dans l'environnement, ou au contraire ne soient trop grosses, avec le risque de rendre la composition abrasive au contact de la peau.

Ainsi, le ratio entre le diamètre moyen équivalent des fibres selon l'invention et la taille moyenne en volume des charges minérales, mesurée selon la méthode d'analyse granulométrique par diffraction laser précitée, est alors avantageusement supérieur ou égal à 10. Ce ratio entre le diamètre moyen équivalent des fibres et la taille moyenne en volume des charges minérales est de préférence inférieur ou égal à 200.

Les fibres selon l'invention peuvent être préparées par des méthodes connues de l'homme du métier. L'on peut par exemple procéder par filage à l'état fondu de la composition polymérique, de manière à obtenir des filaments, lesquels sont ensuite coupés (au moyen d'un dispositif à guillotine ou tout autre moyen connu de l'homme du métier) pour obtenir des fibres ayant la longueur souhaitée.

Il convient de noter que, quelque soit la forme des particules de composition polymérique selon l'invention, l'incorporation des charges minérales dans la matrice polymérique est avantageusement effectuée avant la mise en forme proprement dite du polymère, pour garantir que les charges minérales soient bien dispersées au sein de la matrice de polymère.

Comme expliqué ci-avant, les particules de composition polymérique selon l'invention sont utilisées sous forme d'une dispersion au sein d'une composition cosmétique.

Cette dispersion est réalisée en dispersant lesdites particules dans un fluide porteur, c'est-à-dire un milieu liquide leur servant de véhicule. Ce fluide porteur comprend de l'eau et/ou un ou plusieurs fluides organiques.

Par fluide organique, on désigne selon l'invention des liquides organiques qui peuvent présenter des viscosités très variables. Ainsi, les fluides organiques utilisables dans l'invention peuvent avoir une viscosité dynamique à 20°C allant de 10⁻⁴ à 10³ Pa.s, de préférence de 0,5.10⁻³ à 10² Pa.s.

De tels fluides peuvent être miscibles à l'eau en toutes proportions. Ils peuvent ainsi être choisis parmi les mono-alcools contenant de 2 à 4 atomes de carbone, et les polyols contenant de 2 à 6 atomes de carbone tels qu'en particulier le glycol, le glycérol, le sorbitol.

De tels fluides peuvent également être non miscibles à l'eau, et dans ce cas lorsque la composition contient également de l'eau celle-ci se présente alors sous la forme d'une émulsion. Ils peuvent ainsi être choisis parmi les huiles naturelles ou synthétiques, en particulier les huiles minérales, les huiles végétales, les alcools gras, les acides gras, les esters contenant au moins un acide gras et/ou au moins un alcool gras, et les silicones.

Les alcools et acides gras visés ci-avant sont ceux qui contiennent de 8 à 32, de préférence de 10 à 26, et plus préférentiellement de 12 à 22 atomes de carbone.

Il est bien entendu possible d'utiliser des mélanges de fluides organiques et notamment tous mélanges de n'importe lesquels des fluides décrits ci-avant.

Selon un mode de réalisation particulièrement préféré, le fluide porteur contient de l'eau.

Dans ce cas, la composition cosmétique selon l'invention contient avantageusement au moins 20% en poids d'eau, plus préférentiellement au moins 30% en poids d'eau, et encore plus préférentiellement au moins 50% en poids d'eau, par rapport au poids total de ladite composition.

De manière également préférée, la composition cosmétique selon l'invention contient, en plus de l'eau, un ou plusieurs fluides organiques.

Dans ce cas, la composition cosmétique selon l'invention contient avantageusement au moins 5% en poids de fluide(s) organique(s), plus préférentiellement au moins 10% en poids de fluide(s) organique(s), par rapport au poids total de ladite composition.

La composition cosmétique peut également comprendre tous les ingrédients classiques, connus de l'homme du métier comme entrant dans la composition des produits cométiques pour la peau. Ces ingrédients peuvent être en particulier, et de manière non limitative, choisis parmi : les agents épaississants, les agents tensioactifs, les agents hydratants, les agents de conditionnement de la peau, les filtres UV, les pigments colorés ou non, les agents antioxydants, les agents conservateurs.

Les ingrédients additionnels susceptibles d'être employés dans les compositions selon l'invention peuvent être notamment choisis parmi ceux décrits dans l'International Cosmetic Ingrédient Dictionary and Handbook, régulièrement publié par The Cosmetic, Toiletry, and Fragrance Association.

Selon un mode de réalisation particulièrement avantageux, la composition cosmétique selon l'invention comprend également un ou plusieurs agents actifs anti-rides, différent(s) des charges minérales selon l'invention.

En effet, la Demanderesse constaté un effet de synergie entre les particules de composition polymérique selon l'invention et les agents actifs anti-rides.

De tels agents actifs anti-rides peuvent en particulier être choisis, de manière non limitative, parmi :
- les rétinoides tels que le rétinol, les esters d'acide en C2 à C22 et du rétinol (par exemple le palmitate de rétinyle, l'acétate de rétinyle, le proprionate de rétinyle), le rétinal, les acides rétinoiques ;
- les peptides naturels ou synthétiques, de préférence ceux contenant de 2 à 20 acides aminés et/ou dérivés d'acides aminés, plus préférentiellement de 2 à 10 acides aminés et/ou dérivés d'acides aminés; les dérivés d'acides aminés susceptibles d'être présents dans les oligopeptides sont bien connus de l'homme du métier, et incluent entre autres, les isomères, esters et complexes notamment métalliques de tels acides aminés ;

- les alpha-hydroxyacides et bêta-hydroxyacides (par exemple l'acide glycolique) ;
- les acides cétoniques (par exemple l'acide pyruvique) ;
- l'acide hyaluronique, ses sels (notamment de sodium, de potassium) et ses esters.

Les agents actifs anti-rides peuvent être présents dans des teneurs allant de 0,01 à 10% en poids, et de préférence de 0,1 à 8% en poids, et plus préférentiellement de 0,5 à 5% en poids, par rapport au poids total de la composition cosmétique de l'invention.

La composition cosmétique selon l'invention peut se présenter sous des formes très différentes, telles que en particulier et de manière non limitative des liquides plus ou moins visqueux (comme des fluides, des laits, des sérums), des lotions, des crèmes plus ou moins épaisses, des pâtes, des gels, des mousses, des sprays (compositions pulvérisables).

Elle peut être un produit destiné essentiellement au soin de la peau, et/ou à son maquillage (par exemple une composition de fond de teint, de rouge à lèvre, de fard à joues ou à paupières).

Selon un mode de réalisation particulièrement préféré, la composition selon l'invention se trouve sous forme d'une crème, qui est de préférence constituée d'une émulsion, et plus préférentiellement d'une émulsion huile-dans-eau.

La composition cosmétique selon l'invention peut être préparée par les méthodes connues de l'homme du métier dans le domaine de la préparation de produits cosmétiques. Ces méthodes comprennent en général le mélange des ingrédients de la composition en une ou plusieurs étapes, et peuvent également inclure des étapes de chauffage et/ou de refroidissement.

La présente invention a également pour objet une méthode de traitement cosmétique de la peau, consistant à mettre la peau au contact d'une composition cosmétique telle que décrite ci-avant.

Cette méthode consiste en particulier à appliquer ladite composition cosmétique sur la peau, sur la ou les zones à traiter. Cette application peut être quotidienne, bi-quotidienne (par exemple, le matin et le soir), ou plus épisodique (tous les deux jours, une fois par semaine,....).

Après application sur la peau, la composition peut être soit laissée, soit rincée après un temps de pose qui peut aller de quelques minutes à quelques heures.

La présente demande a enfin pour objet l'utilisation d'une telle composition cosmétique pour prévenir ou diminuer les signes du vieillissement de la peau.

La description détaillée faite ci-avant de la méthode selon l'invention s'applique également à l'utilisation selon l'invention.

Les exemples de réalisation de l'invention ci- sont donnés à titre purement illustratif, et ne sauraient présenter un quelconque caractère limitatif.

### EXEMPLES

### Exemple 1 : réalisation de particules de composition polymérique :

Les matières utilisées sont les suivantes :
- Polymère PA66 de viscosité relative 2,6 ;
- Tourmaline (taille moyenne en volume de particule de 0,8 µm) ;
- Sulfate de baryum (taille moyenne en volume de particule de 0,8 µm) ;
- Dioxyde de titane (taille moyenne en volume de particule de 0,3 µm) ;
- Additif A : Copolymère polyamide/polyoxyde d'alkylène étoile hydrophile obtenu de la façon suivante :
   Dans une autoclave de 7,5 litres équipée d'un agitateur mécanique sont introduits: 1116,0 g d'ε-caprolactame (9,86 mol), 57,6 g d'acide 1,3,5-benzène tricarboxylique (0,27mol), 1826,4 g de Jeffamine M2070 (0,82 mol), 1,9g d'ULTRANOX 236 et 3,5 g d'une solution aqueuse à 50% (p/p) d'acide hypophosphoreux.

Le mélange réactionnel est porté à 250°C sous azote et sous pression atmosphérique et maintenu à cette température pendant 1h. Puis le système est progressivement mis sous vide pendant 30 min jusqu'à une pression de 5 mbars, puis maintenu sous vide pendant une heure supplémentaire. Le systéme est ensuite coulé sur un plateau.
- Polyoxyde d'éthylène de poids moléculaire 400g/mole.

### Préparation de la composition de polymère :

Le polyamide est mélangé à la tourmaline, au sulfate de baryum et au dioxyde de titane de façon à ce que la composition massique finale soit de 70% de PA66, 2,7% de tourmaline, 6,8% de sulfate de baryum et 20,5% de dioxyde de titane. Le mélange est refondu dans une extrudeuse double vis à température de 290°C et extrudé pour obtenir le polymère granulé.

### Préparation de particules de composition polymérique de taille 10 µm :

On introduit dans une extrudeuse double vis 24D de type PRISM des granulés de la composition de polymère obtenue ci-avant à l'aide d'une alimentation volumétrique ainsi qu'un mélange de pastilles de l'additif A (concentration massique de 5%) et de polyoxyde d'éthylène (concentration massique de 19%) l'aide d'une alimentation pondérale. Le mélange est extrudé à un débit fixé entre 2,0 kg /heure. Les températures des différentes plages de l'extrudeuse sont comprises entre 275 et 295°C. La vitesse est fixée à 200 rpm. La pression enregistrée est comprise entre 10 et 13 bars. Les joncs obtenus sont trempés en sortie de filière par un flux d'eau, recueillis dans une panière métallique, égouttés puis séchés.

Les joncs collectés sont ensuite dispersés dans l'eau par simple agitation mécanique. La dispersion ainsi obtenue est tamisée avec un tamis 200 µm pour éliminer les impuretés solides de grande taille telles que des morceaux de jonc non-dispersables. Les rendements pondéraux de récupération de polymère polyamide après tamisage sont supérieurs à 90%. La distribution granulométrique des particules contenues dans la dispersion a été mesurée à l'aide d'un appareil dénommé MasterSize 2000 commercialisé par la société Malvern Instruments. Cette distribution, exprimée en volume, obtenue après application d'Ultrasons, est unimodale et la valeur du pic modal est 10 µm.

L'on obtient ainsi des particules de composition polymérique contenant 70% en poids de PA66 et 30% en poids de charges minérales (dioxyde de titane, sulfate de baryum, tourmaline).

### Exemple 2 : compositions cosmétiques selon l'invention

Les trois compositions ci-dessous ont été préparées, à partir des ingrédients indiqués, pour chacune, dans le tableau correspondant ci-dessous. Pour chaque composition, la teneur de chaque ingrédient est indiquée en pourcentage en poids, par rapport au poids total de la composition.

Les particules de composition polymérique employées sont celles préparées selon l'exemple 1.

### Exemple 2.1 : émulsion anti-âge

| Ingrédients | Teneur en poids |
|---|---|
| *Phase A* | |
| Eau déionisée | 74,1% (qsp 100%) |
| Polymère d'acide acrylique réticulé (commercialisé sous la dénomination Carbopol Ultrez 10 par la société Novéon) | 0,1% |
| Cétéaryl glucoside (commercialisé sous la dénomination Tego Care CG90 par la société Evonik) | 1,0% |
| Glycérine | 3,0% |

| *Phase B* | |
|---|---|
| Stéarate d'éthylhexyle (commercialisé sous la dénomination Tegosoft par la société Evonik) | 6,5% |
| Huile de paraffine | 5,0% |
| Acide stéarique | 1,0% |

| *Phase C* | |
|---|---|
| Oligopeptides de palmitoyle / Céramide 2/ Benzoate d'alkyle C12-C15 / Tribehenin / Stérol de colza PEG 10 (produit commercialisé sous la dénomination | 2,0% |
| Dermaxyl par la société Sederma) | |

| *Phase D* | |
|---|---|
| Eau déionisée | 3,5% |
| Agents conservateurs | 0,3% |

| *Phase E* | |
|---|---|
| Particules de polyamide 66 contenant des charges minérales (exemple 1) | 3,5% |

| *Phase F* | |
|---|---|
| NaOH 10% | q.s. pH = 6 |

Cette composition a été préparée de la manière suivante : le polymère d'acide acrylique a été dispersé dans l'eau, puis après 20 min d'agitation le cétéaryl glucoside et la glycérine ont été ajoutés. Le mélange a alors été chauffé à 75°C.

La phase B a été préparée séparément, en mélangeant ses ingrédients à 75°C.

La phase C a alors été chauffée à 75°C, et ajoutée à la phase B, et le mélange a été homogénéisé à 75°C, puis ajouté à la phase A.

Le mélange résultant a été homogénéisé à 75°C, puis refroidi à 35°C.

Les ingrédients de la phase D ont été pré-mélangés, puis ajoutés au mélange ci-avant.

La phase E (particules de polyamide) a alors été ajoutée progressivement au mélange, sous agitation.

Le pH a été ajusté au moyen de la phase F.

### Exemple 2.2 : lait hydratant pour le visage

| Ingrédients | Teneur en poids |
|---|---|
| *Phase A* | |
| Glycérine à 96% dans l'eau | 9,50% |
| Gomme de xanthane (produit commercialisé sous la dénomination Keltrol CG par la société CP Kelko) | 0,15% |
| Silicate de sodium magnésium lithium (produit commercialisé sous la dénomination Lucentite SWN par la société Kobo Products) | 0,50% |
| Particules de polyamide 66 contenant des charges minérales (exemple 1) | 2,80% |
| EDTA disodique | 0,10% |
| Eau déionisée | qsp 100% |

| *Phase B* | |
|---|---|
| Stéarate de glycéryle (produit commercialisé sous la dénomination Lipomulse 165 par la société Lipo Chemicals) | 5,00% |
| Méthoxycinnamate d'éthylhexyle (produit commercialisé sous la dénomination Parsol MCX par la société Parsol) | 7,50% |
| Alcool stéarylique (produit commercialisé par la société Rita) | 2,00% |
| Alcool cétéarylique 50/50 (produit commercialisé par la société Rita) | 0,75% |
| Stéarate d'isocétyle (produit commercialisé sous la dénomination Crodamol ICS par la société Croda) | 4,00% |
| Cyclopentasiloxane / Cyclohexasiloxane (produit commercialisé sous la dénomination SF1256 par la société Kobo Products) | 4,50% |

| *Phase C* | |
|---|---|
| Triéthanolamine | 2,25% |
| Eau déionisée | 2,00% |

| *Phase D* | |
|---|---|
| Cyclopentasiloxane / Dioxyde de titane / Alumine / PEG-10 diméthicone / Lauryl polyglycéryl-3 polydiméthylsiloxyéthyl diméthicone / Méthicone (produit commercialisé sous la dénomination CM3KG40T7 par la société Kobo Products) | 5,50% |

| *Phase E* | |
|---|---|
| Agents conservateurs (méthyl et propyl parabènes) | 1,25% |

| *Phase F* | |
|---|---|
| Hyaluronate de sodium en solution à 1% dans l'eau | 0,50% |
| Mica / Dioxyde de titane / Polyméthacrylate de méthyle (produit commercialisé sous la dénomination SK-45-R par la société Kobo Products) | 1,00% |
| Parfum | 0,50% |

Cette composition a été préparée de la manière suivante : le silicate de sodium magnésium lithium et l'EDTA disodique sont ajoutés sous agitation à l'eau. La glycérine et la gomme de xanthane sont prémélangées, puis ajoutées au mélange principal sous agitation. Le mélange est alors chauffé à 75-78°C, les particules de polyamide sont ensuite ajoutées et le tout est mélangé pendant 15 minutes.

Les ingrédients de la phase B sont prémélangés à part et chauffés à 78-82°C sous agitation. Puis la phase B est ajoutée à la phase A, et l'ensemble est mélangé sous agitation pendant 15 minutes.

Les ingrédients de la phase C sont prémélangés à part, puis ajoutés au mélange principal après refroidissement de celui-ci à 65°C.

Puis, après refroidissement du mélange principal à 60°C, la phase D est ajoutée, puis après 15 minutes de mélange, la phase E.

Après refroidissement du mélange principal en dessous de 50°C, les ingrédients de la phase F sont ajoutés, puis le mélange est homogénéisé pendant 10 minutes, et la composition résultante est lassée refroidir sous agitation jusqu'à température ambiante.

### Exemple 2.3 : composition antirides

| Ingrédients | Teneur en poids |
|---|---|
| *Phase A* | |
| Cyclopentasiloxane/ PEG/PPG-20/15 Dimethicone (produit commercialisé sous la dénomination SF1528 par la société Kobo Products) | 11,00% |
| Cyclopentasiloxane (produit commercialisé sous la dénomination SF1202 par la société Kobo Products) | 9,00% |
| Cyclopentasiloxane/Diméthicone (produit commercialisé sous la dénomination SF1214 par la société Kobo) | 7,50% |

| *Phase B* | |
|---|---|
| Particules de polyamide 66 contenant des charges minérales (exemple 1) | 7,50% |

| *Phase C* | |
|---|---|
| Glycérine (Glycerin U.S.P. Natural 96%, commercialisé par la société Univar USA Inc.) | 8,00% |
| Chlorure de sodium | 1,00% |
| Butylène glycol/Eau/Palmitoyl hydroxypropyltrimonium, amylopectine/Polymère de glycérine/Polysorbate20/ Rétinol/Phénoxyéthanol/Parabènes/Lécithine hydrogénée/BHT/BHA (produit commercialisé sous la dénomination Gs-VA100C par la société Kobo Products) | 0,50% |
| Eau/Papaine /Palmitoyl hydroxypropyltrimonium amylopectine/Polymère réticulé de glycérine/ Phénoyéthanol/Lécithine hydrogénée /Parabènes (produit commercialisé sous la dénomination GsPPY par la société Kobo Products) | 0,50% |
| Polysorbate 80 (produit commercialisé sous la dénomination Liposorb O-20 par la société LIPO Chemicals) | 0,20% |
| Quaternium-15 (produit commercialisé sous la dénomination Dowicil 200 par la société DOW Chemical) | 0,10% |
| Eau déionisée | qsp 100% |

Cette composition a été préparée de la manière suivante : les composés de la phase A ont été mélangés, et le mélange homogénéisé pendant 15 minutes. Puis les particules de polymère (phase B) ont été ajoutées, et l'homogénéisation poursuivie pendant 15 minutes.

Les ingrédients de la phase C ont été pré-mélangés à part, puis ajoutées progressivement au mélange principal en cinq portions, en respectant un temps de mélange de 15-20 minutes entre chaque ajout.

Après homogénéisation complète du mélange, la composition ainsi obtenue a alors été conditionnée, en la versant dans des récipients appropriés.

## Revendications

1. Composition cosmétique, comprenant des particules d'une composition polymérique laquelle contient une matrice polymérique et au moins deux charges minérales de types différents choisis parmi les oxydes, les sulfates, les carbonates, les phosphates et les silicates, uniformément dispersées dans la matrice polymérique, ayant des propriétés d'absorption et/ou d'émission dans la région d'infrarouge lointain allant de 2 µm à 20 µm, lesdites particules de composition polymérique étant dispersées au sein d'un fluide porteur comprenant de l'eau et/ou un ou plusieurs fluides organiques.

2. Composition selon la revendication précédente, **caractérisée en ce que** la matrice polymérique est choisie dans le groupe comprenant les polyesters, les polyoléfines, les polymères à base de cellulose-ester, les polymères et copolymères acryliques, les polyamides, leurs copolymères et leurs mélanges.

3. Composition selon la revendication précédente, **caractérisée en ce que** la matrice polymérique est constituée de polyamide, de préférence choisi entre le polyamide 6, le polyamide 66 et les copolymères de polyamide 6/polyamide 66 en toutes proportions.

4. Composition selon l'une quelconque des revendications précédentes, **caractérisée en ce que** la composition polymérique contient au moins trois charges minérales de types différents, choisis parmi les types suivants : les oxydes, les sulfates, les carbonates, les phosphates et les silicates.

5. Composition selon l'une quelconque des revendications précédentes, **caractérisée en ce que** la composition polymérique contient au moins deux charges minérales de types différents, et de préférence au moins trois charges minérales de types différents, choisis parmi les types suivants : les oxydes, les sulfates, et les silicates, et de préférence parmi le dioxyde de titane, un sulfate de métal alcalin ou alcalino-terreux et un silicate, et de manière encore plus préférée parmi le dioxyde de titane, le sulfate de baryum, et la tourmaline.

6. Composition selon la revendication précédente, **caractérisée en ce que** la composition polymérique contient trois charges minérales de types différents, qui sont un oxyde, un sulfate, et un silicate, et plus préférentiellement l'association dioxyde de titane/sulfate de baryum/tourmaline.

7. Composition selon la revendication 1, **caractérisée en ce que** la composition polymérique contient au moins deux charges minérales de types différents, et de préférence au moins trois charges minérales de types différents choisies parmi les types suivants : les oxydes, les phosphates, et les silicates.

8. Composition selon la revendication précédente, **caractérisée en ce que** la composition polymérique contient trois charges minérales de types différents, qui sont un oxyde, un phosphate, et un silicate.

9. Composition selon l'une quelconque des revendications précédentes, **caractérisée en ce que** la proportion en poids de charge(s) minérale(s) par rapport au poids total de la composition polymérique est supérieure ou égale à 1,0 %, de préférence supérieure ou égale à 1,5 % et plus préférentiellement encore supérieure ou égale à 2,5 %.

10. Composition selon l'une quelconque des revendications précédentes, **caractérisée en ce que** la proportion en poids de charge(s) minérale(s) par rapport au poids total de la composition polymérique est inférieure ou égale à 50 %, de préférence inférieure ou égale à 40%, avantageusement inférieure ou égale à 30 %.

11. Composition selon l'une quelconque des revendications précédentes, **caractérisée en ce que** la ou les charges minérales se trouvent sous forme de particules présentant une taille moyenne en volume, mesurée selon la méthode d'analyse granulométrique par diffraction laser, inférieure ou égale à 2 µm, de préférence allant de 0,1 à 2 µm, plus préférentiellement de 0,2 à 1,5 µm, et encore plus préférentiellement de 0,2 à 1 µm.

12. Composition selon l'une quelconque des revendications précédentes, **caractérisée en ce que** les particules de composition polymérique sont présentes en une teneur allant de 0,5 à 20% en poids, de préférence de 1 à 15% en poids, et plus préférentiellement de 2 à 10% en poids, par rapport au poids total de la composition cosmétique.

13. Composition selon l'une quelconque des revendications précédentes, **caractérisée en ce que** le ou les fluide(s) organique(s) est (sont) choisi(s) parmi :
- les mono-alcools contenant de 2 à 4 atomes de carbone, et les polyols contenant de 2 à 6 atomes de carbone ;
- les huiles minérales, les huiles végétales, les alcools gras, les acides gras, les esters contenant au moins un acide gras et/ou au moins un alcool gras, et les silicones ;
- et leurs mélanges.

14. Composition selon l'une quelconque des revendications précédentes, **caractérisée en ce qu'**elle contient au moins 20% en poids d'eau, de préférence au moins 30% en poids d'eau, et encore plus préférentiellement au moins 50% en poids d'eau, par rapport au poids total de la composition.

15. Composition selon la revendication précédente, **caractérisée en ce qu'**elle contient en outre au moins 5% en poids de fluide(s) organique(s), de préférence au moins 10% en poids de fluide(s) organique(s), par rapport au poids total de la composition.

16. Composition selon l'une quelconque des revendications précédentes, **caractérisée en ce que** les particules de composition polymérique présentent une forme sensiblement sphérique, et présentent une taille moyenne en volume, mesurée selon la méthode d'analyse granulométrique par diffraction laser, de préférence inférieure ou égale à 250 µm, plus préférentiellement allant de 5 à 150 µm, et encore plus préférentiellement de 10 à 50 µm.

17. Composition selon l'une quelconque des revendications 1 à 15, **caractérisée en ce que** les particules de composition polymérique présentent la forme de fibres, dont la longueur moyenne est de préférence inférieure ou égale à 3 mm, plus préférentiellement inférieure ou égale à 2 mm, encore plus préférentiellement inférieure ou égale à 1,5 mm.

18. Composition selon l'une quelconque des revendications précédentes, **caractérisée en ce qu'**elle comprend également un ou plusieurs agent(s) actif(s) anti-rides, différent(s) des charges minérales selon l'invention, de préférence choisi(s) parmi :
- les rétinoides tels que le rétinol, les esters d'acide en C2 à C22 et du rétinol, le rétinal, les acides rétinoiques ;
- les peptides naturels ou synthétiques, de préférence ceux contenant de 2 à 20 acides aminés et/ou dérivés d'acides aminés, plus préférentiellement de 2 à 10 acides aminés et/ou dérivés d'acides aminés;
- les alpha-hydroxyacides et bêta-hydroxyacides ;
- les acides cétoniques;
- l'acide hyaluronique, ses sels et ses esters.

19. Composition selon la revendication précédente, **caractérisée en ce qu'**elle contient ledit ou lesdits agent(s) actif(s) anti-rides dans des teneurs allant de 0,01 à 10% en poids, de préférence de 0,1 à 8% en poids, et plus préférentiellement de 0,5 à 5% en poids, par rapport au poids total de la composition.

20. Méthode de traitement cosmétique de la peau, consistant à mettre la peau au contact d'une composition cosmétique telle que définie dans l'une quelconque des revendications précédentes.

21. Utilisation d'une composition cosmétique telle que définie dans l'une quelconque des revendications 1 à 19, pour prévenir ou diminuer les signes du vieillissement de la peau.

## Patentansprüche

1. Kosmetikzusammensetzung, umfassend Teilchen einer Polymerzusammensetzung, die eine Polymermatrix und mindestens zwei mineralische Füllstoffe unterschiedlicher Typen, ausgewählt aus Oxiden, Sulfaten, Carbonaten, Phosphaten und Silikaten, umfasst, die gleichmäßig in der Polymermatrix dispergiert sind, mit Absorptions- und/oder Emissionseigenschaften im fernen Infrarotbereich von 2 µm bis 20 µm, wobei die Teilchen der Polymerzusammensetzung in einer Trägerflüssigkeit dispergiert sind, die Wasser und/oder eine oder mehrere organische Flüssigkeiten umfasst.

2. Zusammensetzung nach dem vorhergehenden Anspruch, **dadurch gekennzeichnet, dass** die Polymermatrix aus der Gruppe ausgewählt ist, bestehend aus Polyestern, Polyolefinen, Polymeren auf Celluloseesterbasis, Acrylpolymeren und -copolymeren, Polyamiden, deren Copolymeren und deren Gemischen.

3. Zusammensetzung nach dem vorhergehenden Anspruch, **dadurch gekennzeichnet, dass** die Polymermatrix aus Polyamid besteht, das vorzugsweise aus Polyamid 6, Polyamid 66 und Copolymeren von Polyamid 6/Polyamid 66 in beliebigen Verhältnissen ausgewählt ist.

4. Zusammensetzung nach einem der vorhergehenden Ansprüche, **dadurch gekennzeichnet, dass** die Polymerzusammensetzung mindestens drei mineralische Füllstoffe unterschiedlicher Typen, ausgewählt aus den folgenden Typen: Oxiden, Sulfaten, Carbonaten, Phosphaten und Silikaten, umfasst.

5. Zusammensetzung nach einem der vorhergehenden Ansprüche, **dadurch gekennzeichnet, dass** die Polymerzusammensetzung mindestens zwei mineralische Füllstoffe unterschiedlicher Typen und vorzugsweise mindestens drei mineralische Füllstoffe unterschiedlicher Typen, ausgewählt aus den folgenden Typen: Oxiden, Sulfaten und Silikaten und vorzugsweise aus Titandioxid, einem Alkalimetall- oder Erdalkalimetallsulfat und einem Silikat und noch stärker bevorzugt aus Titandioxid, Bariumsulfat und Turmalin, umfasst.

6. Zusammensetzung nach dem vorhergehenden Anspruch, **dadurch gekennzeichnet, dass** die Polymerzusammensetzung drei mineralische Füllstoffe unterschiedlicher Typen umfasst, bei denen sich um ein Oxid, ein Sulfat und ein Silikat und stärker bevorzugt um die Kombination Titandioxid/Bariumsulfat/Turmalin handelt.

7. Zusammensetzung nach Anspruch 1, **dadurch gekennzeichnet, dass** die Polymerzusammensetzung mindestens zwei mineralische Füllstoffe unterschiedlicher Typen und vorzugsweise mindestens drei mineralische Füllstoffe unterschiedlicher Typen, ausgewählt aus den folgenden Typen: Oxiden, Phosphaten und Silikaten, umfasst.

8. Zusammensetzung nach dem vorhergehenden Anspruch, **dadurch gekennzeichnet, dass** die Polymerzusammensetzung drei mineralische Füllstoffe unterschiedlicher Typen umfasst, bei denen es sich um ein Oxid, ein Phosphat und ein Silikat handelt.

9. Zusammensetzung nach einem der vorhergehenden Ansprüche, **dadurch gekennzeichnet, dass** der Gewichtsanteil an mineralische(m/n) Füllstoff(en), bezogen auf das Gesamtgewicht der Polymerzusammensetzung, größer als oder gleich 1,0%, vorzugsweise größer als oder gleich 1,5% und noch stärker bevorzugt größer als oder gleich 2,5% ist.

10. Zusammensetzung nach einem der vorhergehenden Ansprüche, **dadurch gekennzeichnet, dass** der Gewichtsanteil an mineralische(m/n) Füllstoff(en), bezogen auf das Gesamtgewicht der Polymerzusammensetzung, kleiner als oder gleich 50%, vorzugsweise kleiner als oder gleich 40%, vorteilhafterweise kleiner als oder gleich 30% ist.

11. Zusammensetzung nach einem der vorhergehenden Ansprüche, **dadurch gekennzeichnet, dass** der oder die mineralische(n) Füllstoff(e) in Form von Teilchen vorliegt, die eine gemäß dem Laserbeugungsteilchengrößenanalyseverfahren gemessene volumenmittlere Größe von weniger als oder gleich 2 µm, vorzugsweise von 0,1 bis 2 µm, stärker bevorzugt von 0,2 bis 1,5 µm und noch stärker bevorzugt von 0,2 bis 1 µm aufweisen.

12. Zusammensetzung nach einem der vorhergehenden Ansprüche, **dadurch gekennzeichnet, dass** die Teilchen der Polymerzusammensetzung in einem Gehalt von 0,5 bis 20 Gew.-%, vorzugsweise von 1 bis 15 Gew.-% und stärker bevorzugt von 2 bis 10 Gew.-%, bezogen auf das Gesamtgewicht der Kosmetikzusammensetzung, vorliegen.

13. Zusammensetzung nach einem der vorhergehenden Ansprüche, **dadurch gekennzeichnet, dass** die organische Flüssigkeit oder die organischen Flüssigkeiten aus den folgenden ausgewählt ist/sind:
- Monoalkoholen mit 2 bis 4 Kohlenstoffatomen und Polyolen mit 2 bis 6 Kohlenstoffatomen,
- Mineralölen, Pflanzenölen, Fettalkoholen, Fettsäuren, Estern, die mindestens eine Fettsäure und/oder mindestens einen Fettalkohol enthalten, und Silikonen
- und deren Gemischen.

14. Zusammensetzung nach einem der vorhergehenden Ansprüche, **dadurch gekennzeichnet, dass** sie mindestens 20 Gew.-% Wasser, vorzugsweise mindestens 30 Gew.-% Wasser und noch stärker bevorzugt mindestens 50 Gew.-% Wasser, bezogen auf das Gesamtgewicht der Zusammensetzung, enthält.

15. Zusammensetzung nach dem vorhergehenden Anspruch, **dadurch gekennzeichnet, dass** sie außerdem mindestens 5 Gew.-% organische Flüssigkeit(en), vorzugsweise mindestens 10 Gew.-% organische Flüssigkeit(en), bezogen auf das Gesamtgewicht der Zusammensetzung, enthält.

16. Zusammensetzung nach einem der vorhergehenden Ansprüche, **dadurch gekennzeichnet, dass** die Teilchen der Polymerzusammensetzung eine im Wesentlichen sphärische Form aufweisen und eine gemäß dem Laserbeugungsteilchengrößenanalyseverfahren gemessene volumenmittlere Größe von vorzugsweise weniger als oder gleich 250 µm, stärker bevorzugt von 5 bis 150 µm und noch stärker bevorzugt von 10 bis 50 µm aufweisen.

17. Zusammensetzung nach einem der Ansprüche 1 bis 15, **dadurch gekennzeichnet, dass** die Teilchen der Polymerzusammensetzung die Form von Fasern aufweisen, deren mittlere Länge vorzugsweise weniger als oder gleich 3 mm, stärker bevorzugt weniger als oder gleich 2 mm, noch stärker bevorzugt weniger als oder gleich 1,5 mm beträgt.

18. Zusammensetzung nach einem der vorhergehenden Ansprüche, **dadurch gekennzeichnet, dass** sie außerdem einen oder mehrere Antifaltenwirkstoff(e) enthält, der/die von den erfindungsgemäßen mineralischen Füllstoffen verschieden ist/sind, vorzugsweise ausgewählt aus:
- Retinoiden, wie Retinol, Estern von C2- bis C22-Säure und Retinol, Retinal, Retinsäuren,
- natürlichen oder synthetischen Peptiden, vorzugsweise solchen, die 2 bis 20 Aminosäuren und/oder Aminosäurederivate, stärker bevorzugt 2 bis 10 Aminosäuren und/oder Aminosäurederivate enthalten,
- alpha-Hydroxysäuren und beta-Hydroxysäuren,
- Ketonsäuren,
- Hyaluronsäure, deren Salzen und deren Estern.

19. Zusammensetzung nach dem vorhergehenden Anspruch, **dadurch gekennzeichnet, dass** sie den oder die Antifaltenwirkstoff(e) in Gehalten von 0,01 bis 10 Gew.-%, vorzugsweise von 0,1 bis 8 Gew.-% und stärker bevorzugt von 0,5 bis 5 Gew.-%, bezogen auf das Gesamtgewicht der Zusammensetzung, enthält.

20. Kosmetisches Behandlungsverfahren für die Haut, bei dem man die Haut mit einer Kosmetikzusammensetzung nach einem der vorhergehenden Ansprüche in Kontakt bringt.

21. Verwendung einer Kosmetikzusammensetzung nach einem der Ansprüche 1 bis 19 zur Verhinderung oder Verringerung der Anzeichen von Alterung der Haut.

## Claims

1. A cosmetic composition, comprising particles of a polymeric composition which contains a polymer matrix and at least two mineral fillers of different types chosen from the following types: oxides, sulfates, carbonates, phosphates and silicates, uniformly dispersed in the polymer matrix, having properties of absorption and/or emission in the far infrared region ranging from 2 µm to 20 µm, said particles of polymeric composition being dispersed in a carrier fluid comprising water and/or one or more organic fluids.

2. The composition as claimed in the preceding claim, **characterized in that** the polymer matrix is chosen from the group comprising polyesters, polyolefins, polymers based on cellulose esters, acrylic polymers and copolymers, polyamides, copolymers thereof and blends thereof.

3. The composition as claimed in the preceding claim, **characterized in that** the polymer matrix consists of polyamide, preferably chosen from polyamide 6, polyamide 66 and copolymers of polyamide 6/polyamide 66 in any proportions.

4. The composition as claimed in any one of the preceding claims, **characterized in that** the polymeric composition contains at least three mineral fillers of different types, chosen from the following types: oxides, sulfates, carbonates, phosphates and silicates.

5. The composition as claimed in the preceding claim, **characterized in that** the polymeric composition contains at least two mineral fillers of different types, and preferably at least three mineral fillers of different types, chosen from the following types: oxides, sulfates and silicates, and preferably from titanium dioxide, an alkali metal or alkaline-earth metal sulfate and a silicate, and even more preferably from titanium dioxide, barium sulfate and tourmaline.

6. The composition as claimed in the preceding claim, **characterized in that** the polymeric composition contains three mineral fillers of different types, which are an oxide, a sulfate and a silicate, and more preferentially the titanium dioxide/barium sulfate/tourmaline combination.

7. The composition as claimed in claim 1, **characterized in that** the polymeric composition contains at least two mineral fillers of different types, and preferably at least three mineral fillers of different types, chosen from the following types: oxides, phosphates and silicates.

8. The composition as claimed in the preceding claim, **characterized in that** the polymeric composition contains three mineral fillers of different types, which are an oxide, a phosphate and a silicate.

9. The composition as claimed in any one of the preceding claims, **characterized in that** the weight proportion of mineral filler(s) relative to the total weight of the polymeric composition is greater than or equal to 1.0%, preferably greater than or equal to 1.5% and even more preferentially greater than or equal to 2.5%.

10. The composition as claimed in any one of the preceding claims, **characterized in that** the weight proportion of mineral filler(s) relative to the total weight of the polymeric composition is less than or equal to 50%, preferably less than or equal to 40% and advantageously less than or equal to 30%.

11. The composition as claimed in any one of the preceding claims, **characterized in that** the mineral filler(s) is (are) in the form of particles which have a volume-average size, measured according to the laser diffraction particle size analysis method, of less than or equal to 2 µm, preferably ranging from 0.1 to 2 µm, more preferentially from 0.2 to 1.5 µm and even more preferentially from 0.2 to 1 µm.

12. The composition as claimed in any one of the preceding claims, **characterized in that** the particles of polymeric composition are present in a content ranging from 0.5% to 20% by weight, preferably from 1% to 15% by weight and more preferentially from 2% to 10% by weight, relative to the total weight of the cosmetic composition.

13. The composition as claimed in any one of the preceding claims, **characterized in that** the organic fluid(s) is (are) chosen from:
- monoalcohols containing from 2 to 4 carbon atoms and polyols containing from 2 to 6 carbon atoms;
- mineral oils, vegetable oils, fatty alcohols, fatty acids, esters containing at least one fatty acid and/or at least one fatty alcohol, and silicones;
- and mixtures thereof.

14. The composition as claimed in any one of the preceding claims, **characterized in that** it contains at least 20% by weight of water, preferably at least 30% by weight of water and even more preferentially at least 50% by weight of water, relative to the total weight of the composition.

15. The composition as claimed in the preceding claim, **characterized in that** it also contains at least 5% by weight of organic fluid(s), preferably at least 10% by weight of organic fluid(s), relative to the total weight of the composition.

16. The composition as claimed in any one of the preceding claims, **characterized in that** the particles of polymeric composition have a substantially spherical shape, and have a volume-average size, measured according to the laser diffraction particle size analysis method, preferably of less than or equal to 250 µm, more preferentially ranging from 5 to 150 µm and even more preferentially from 10 to 50 µm.

17. The composition as claimed in any one of claims 1 to 15, **characterized in that** the particles of polymeric composition have the shape of fibers, the average length of which is preferably less than or equal to 3 mm, more preferentially less than or equal to 2 mm and even more preferentially less than or equal to 1.5 mm.

18. The composition as claimed in any one of the preceding claims, **characterized in that** it also comprises one or more antiwrinkle active agent(s) different from the mineral fillers according to the invention, preferably chosen from:
- retinoids, such as retinol, esters of a C₂ to C₂₂ acid and of retinol, retinal, retinoic acids;
- natural or synthetic peptides, preferably those containing from 2 to 20 amino acids and/or amino acid derivatives, more preferentially from 2 to 10 amino acids and/or amino acid derivatives;
- alpha-hydroxy acids and beta-hydroxy acids;
- ketone acids;
- hyaluronic acid, salts thereof and esters thereof.

19. The composition as claimed in the preceding claim, **characterized in that** it contains said antiwrinkle active agent(s) in contents ranging from 0.01% to 10% by weight, preferably from 0.1% to 8% by weight and more preferentially from 0.5% to 5% by weight, relative to the total weight of the composition.

20. A cosmetic treatment method for the skin, consisting in bringing the skin into contact with a cosmetic composition as defined in any one of the preceding claims.

21. The use of a cosmetic composition as defined in any one of claims 1 to 19, for preventing or reducing the signs of skin aging.
